# EUROPEAN PATENT SPECIFICATION

(11) **EP 2 968 882 B1**
(45) Date of publication and mention of the grant of the patent: **19.05.2021**
(21) Application number: 14779965.4
(22) Date of filing: 12.03.2014
(51) Int. Cl.: A61K 31/5575, A61P 27/02, A61K 47/02, A61M 31/00

(54) **DRUG DELIVERY DEVICE COMPRISING SILICON-BASED CARRIER PARTICLES**
ARZNEIMITTELABGABEVORRICHTUNG MIT TRÄGERTEILCHEN AUF SILICIUMBASIS
DISPOSITIF D'ADMINISTRATION DE MÉDICAMENT COMPRENANT DES PARTICULES DE TRANSPORT À BASE DE SILICIUM

(30) Priority: 12.03.2013 US 201361778111 P
(43) Date of publication of application: 20.01.2016
(73) Proprietor: EyePoint Pharmaceuticals US, Inc., Watertown, MA 02472 (US); PSIMedica Limited, Worcestershire, WR14 3SZ (GB)
(72) Inventor: CANHAM, Leigh, T., Malvern Worcestershire WR14 3LR (GB); GUO, Hong, Wayland, MA 01778 (US); NAZZARO, Martin, Quincy, MA 02169 (US); BARNETT, Christian, Pershore Worcestershire WR10 2JX (GB)
(74) Representative: Vossius & Partner Patentanwälte Rechtsanwälte mbB
(86) International application number: PCT/US2014/024362
(87) International publication number: WO 2014/165097

(56) References cited:
- EP-A2- 0 092 708
- EP-B1- 1 140 027
- WO-A1-2010/111627
- WO-A1-2012/061377
- WO-A2-2012/088306
- US-A1- 2010 196 435
- US-A1- 2012 207 682
- US-A1- 2013 053 794

## Description

### BACKGROUND

There has been considerable interest within the pharmaceutical industry in the development of dosage forms that provide controlled release of therapeutic agents over a period of time. Releasing an active substance in this way can help to improve bioavailability and ensure that appropriate concentrations of the agent are provided for a sustained period without the need for repeated dosing. In turn, this also helps to minimize the effects of patient non-compliance which is frequently an issue with other forms of administration.

With conventional dosing (tablets, injections, etc.), the concentration of drug in a given area of the body increases through an ineffective concentration and eventually reaches an effective concentration. Frequently the concentration may actually reach some toxic threshold. After a relatively short period, however, the drug concentration decreases as drug is either metabolized in the body or is eliminated. Frequently, drug levels decrease so low that therapeutic levels are no longer maintained. A second dose is then given and the cycle is repeated. The goal of a sustained release system is to maintain drug levels within the therapeutic range and ideally at a constant level US 2013/053794 describes an ocular implant providing sustained release of latanoprost.

However, many of currently available sustained release systems fall short of one or more of these goals. Certain sustained release systems lose efficacy over time due to degradation, dispersal within the body, or other unwanted biological processes. Some sustained release systems deliver the drug over too short a time scale, necessitating frequent replacement. Still other sustained release systems perform adequately for releasing one drug, but are not readily customizable to deliver a variety of different classes of therapeutics. Therefore, there is a continuing need for the development of improved dosage forms for the controlled release of therapeutic agents.

### SUMMARY

The present invention is defined by the claims. Accordingly, the present invention relates to a device for delivering a beneficial substance to an eye of a patient, comprising a shell and a plurality of particles disposed within the shell, the particles comprising a porous silicon-based carrier material and a beneficial substance disposed within pores of the carrier material, and the shell comprising at least one portion that is permeable to the beneficial substance, wherein the beneficial substance is selected from the group consisting of neuroprotectants, antibacterials, antiallergenics, antiinflammatories, decongestants, miotics, mydriatics, and sympathomimetics. Likewise, the present invention relates to a method of making a device, comprising: (a) providing a tube having first and second ends; (b) inserting a plurality of particles into the tube, wherein the particles comprise a porous silicon-based carrier material; (c) adding a first member to the first end of the tube before or after inserting the plurality of particles into the tube; and (d) adding a second member to the second end to the tube before or after inserting the plurality of particles into the tube, wherein at least one of the tube, the first member, and the second member is permeable to the beneficial substance.

This specification generally provides a device for delivering a beneficial substance to a patient. The device comprises a shell enclosing a plurality of carrier particles. The carrier particles are porous, and a beneficial substance, such as a drug, is disposed in the pores. The drug can diffuse out of the pores, into the interior of the device, and then through at least one permeable portion of the shell. This specification also provides methods of making and using these devices.

The devices described herein have several advantageous properties. For instance, because each device is administered as a single unit, the device makes it easier for a user to administer a fixed dose. Thus, the device may reduce the chance of accidentally administering an incorrect dose and the chance of an end-user measuring a dose inaccurately. The device can also advantageously localize administration of the beneficial substance to a desired area in a patient's body, because the shell contains the particles at a desired region of the body. In addition, the device comprises a shell that advantageously protects the carrier and beneficial substance against unwanted reactions in the body. For instance, the shell may protect the carrier and beneficial substance against enzymatic degradation or the patient's antibodies. The shell can also protect its contents from cells that would adhere to, break down, or otherwise modify the carrier or beneficial substance. The device may provide improved protection to the beneficial substance during the manufacturing process. For instance, the carrier particles and/or shell can stabilize the beneficial substance during the manufacturing process, for instance during steps that involve heat. The device can also provide improved protection of the beneficial substance during storage, for instance by stabilizing the beneficial substance from interaction with air or humidity. Furthermore, the device can stabilize the beneficial substance over the course of treatment.

In certain aspects, the present disclosure also provides a device comprising a shell and a plurality of particles disposed within the shell, the particles comprising a porous carrier material and a beneficial substance disposed within pores of the carrier, and the shell comprising at least one portion that is permeable to the beneficial substance.

In some aspects, the disclosure also provides a method of making a device, comprising: (a) providing a tube having first and second ends; (b) inserting a plurality of particles into the tube, wherein the particles comprise porous silicon-based carrier material; (c) adding a first member to the first end of the tube; and (d) adding a second member to the second end to the tube, wherein at least one of the tube, the first member, and the second member is permeable to the beneficial substance. In some aspects, the disclosure also provides a device made by this method. Step (b) may be performed before step (c), and also step (c) may be performed before step (b).

In certain aspects, the disclosure provides a method of delivering a beneficial substance to a patient, comprising administering to a patient a device as described herein, whereby the beneficial substance is released from the device into the patient after administration.

The shell may comprise a polymer. The shell may comprise: a tube having first and second ends; a first member positioned at the first end; and a second member positioned at the second end; wherein the particles are inside the tube and the first and second members retain the particles in the tube.

The tube may have a substantially cylindrical shape. The tube may be impermeable to the beneficial substance. For instance, the tube may comprise poly(lactic-co-glycolic acid) (PLGA). The first member and second member may be permeable to the beneficial substance. For instance, the first member and second member may both comprise poly(vinyl alcohol) (PVA). The first member may be permeable to the beneficial substance and the second member may be impermeable to the beneficial substance. For instance, the first member may comprise poly(vinyl alcohol) (PVA). The second member may comprise silicone.

The tube may have a length of between 1 and 4 mm. The tube may have a diameter of between 0.2 - 0.5 mm.

The carrier material may be mesoporous. The carrier material may have a porosity in the range of about 30 to about 90%, for instance about 50% to about 80% or about 70% to about 90%. The carrier material may be silicon-based. The carrier material may comprise elemental silicon. In some embodiments, the carrier material comprises silica. The pores in the carrier material may be substantially parallel. The carrier material may comprise anodized silicon. The carrier material may be resorbable or bio-erodible.

The particles may have a largest dimension, on the average, of between about 1 micron and about 20 microns. The device may release the beneficial substance at a release rate, the release rate being determined primarily by release of the beneficial substance from pores of the carrier material. When the device is placed in a biological medium, the beneficial substance may be released from the device according to a substantially zero-order release profile.

The beneficial substance may be selected from small molecules, proteins, peptides, antibodies, carbohydrates, lipids, polymers, oligonucleotides, and polynucleotides. The beneficial substance may be ranibizumab or bevacizumab.

The device may be produced using a method in which the particles comprise a beneficial substance disposed within pores of the carrier material. The method may further comprise contacting the device to a beneficial substance and allowing the beneficial substance to enter pores of the carrier material. Adding the first member may comprise contacting the first end of the tube to a polymer solution and curing the polymer solution, thereby forming a first member on the first end of the tube. Adding the second member may comprise contacting the second end of the tube to a polymer solution and curing the polymer solution, thereby forming a second member on the second end of the tube. The first and second ends may be cured simultaneously.

The device may release the beneficial substance for between about one month and one year when immersed in simulated body fluid. Administering the device may comprise injecting, implanting, or inserting the device into the patient.

### BRIEF DESCRIPTION OF THE DRAWINGS

The devices will now be described in more detail with reference to preferred aspects, given only by way of example, and with reference to the accompanying drawings, in which:
Figure 1 is an enlarged exploded perspective drawing of a cylindrical device for delivering a beneficial substance.
Figure 2 is an enlarged exploded perspective drawing of a device for delivering a beneficial substance, made with a tube with a square cross-section.
Figure 3 is an enlarged perspective drawing of a cylindrical device for delivering a beneficial substance.
Figure 4 is an enlarged cross-sectional view of a device for delivering a beneficial substance, in which the beneficial substance diffuses out of one end of the device.
Figure 5 is an enlarged cross-sectional view of a device for delivering a beneficial substance, in which the beneficial substance diffuses out of both ends of the device.

### DETAILED DESCRIPTION

### 1. Devices comprising porous carrier particles

This specification provides a device for delivering a beneficial substance to a patient. The device comprises a shell enclosing a plurality of carrier particles. The carrier particles are porous, and a beneficial substance, such as a drug, is disposed in the pores. The beneficial substance can diffuse out of the pores, into the interior of the device, and then through at least one permeable portion of the shell. This specification also provides methods of making and using these devices.

### A. Characteristics of the shell

The shell encloses the carrier particles, keeping the carrier localized in one area of the body. At the same time, the shell allows the beneficial substance (such as a drug) to exit the shell and reach target tissues. Thus, at least a portion of the shell is permeable to the beneficial substance.

The shell can also provide structure to the device. The shell may be dimensionally stable and retain its shape in the absence of the particles. For instance, the shell may comprise a rigid tube that retains its shape even when not filled with the carrier material particles. The tube may also retain its shape in the absence of the first end and second end of the shell.

The device may comprise a single shell.

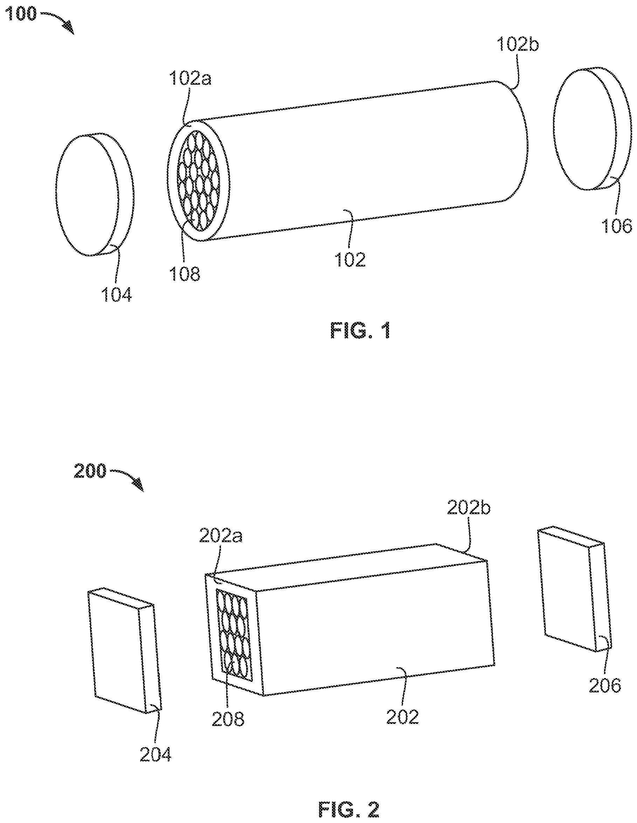

The shell may be made from a tube with a member closing each end, so that the members hold the particles inside. A device 100 of this type is illustrated in Fig. 1, in exploded form. The tube 102 has a first end 102a and a second end 102b. A first member 104 contacts the first end 102a of the tube 102. A second member 106 contacts the second end 102b of the tube 102. The tube 102, first member 104, and second member 106 enclose a plurality of porous particles 108. The pores are suitable for receiving a beneficial substance like a drug.

While, in Figure 1, the first and second members 104 and 106 are shown having about the same thickness, they may also be given different thicknesses. In addition, in this illustration the first and second members 104 and 106 have approximately the same diameter as the tube 102. However, the members may have a slightly larger or slightly smaller diameter as long as they are appropriately sized to retain the particles in the tube. As will be discussed in more detail in Section 2, the first and second members may be formed *in situ* at the ends of the tube, by inducing cross-linking of a polymeric layer at the ends of the tube.

Although the device of Figure 1 is substantially cylindrical, (i.e., it has a tube with a circular cross-section) other geometries are possible. The tube can have a cross-section that is a circle, an oval, a square, a rectangle, a hexagon, or any other shape that is suitable for containing particles inside. Figure 2 illustrates an exploded view of a device 200 made with a tube 202 having a square cross section. The tube 202 has a first end 202a and a second end 202b. A first member 204 contacts the first end 202a, and a second member 206 contacts the second end 202b. The tube 202, first member 204, and second member 206 enclose a plurality of porous particles 108.

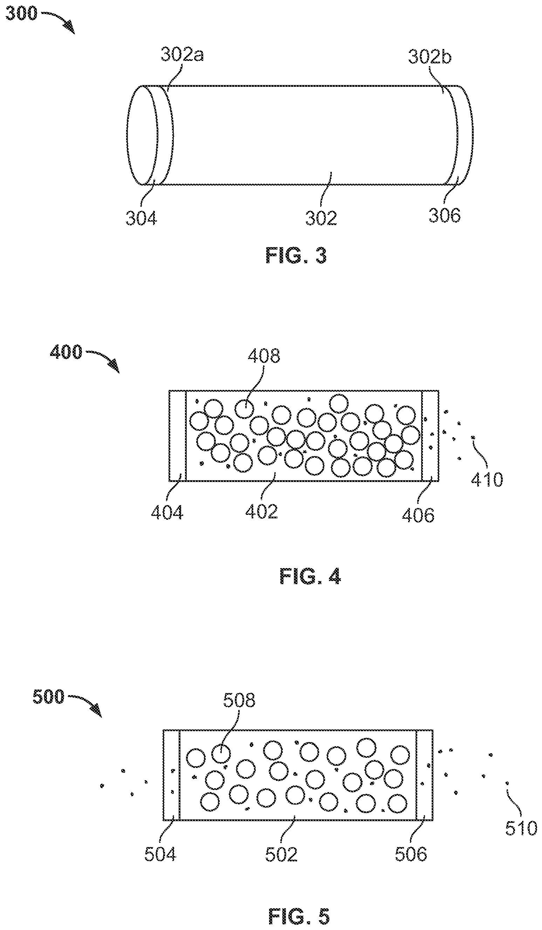

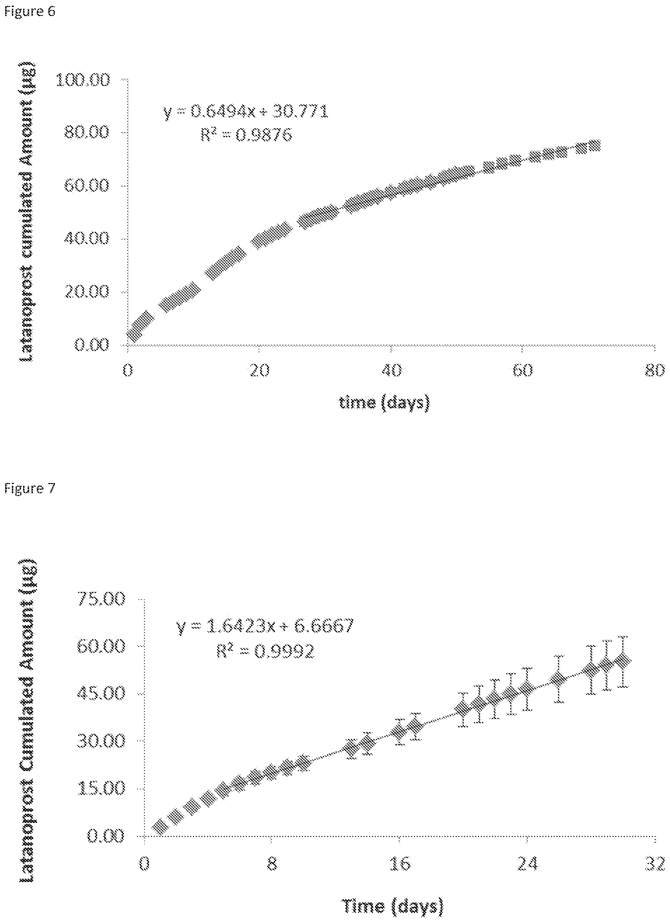

Figure 3 illustrates a device 300 with the first and second members in contact with the tube. In particular, the tube 302 has a first end 302a in contact with a first member 304. The tube 302 has a second end 302b in contact with a second member 306.

The length of the device (i.e., the distance between the first member and the second member) may be from about 1 - 2 mm, 2 - 4 mm, 4 - 6 mm, 6 - 8 mm, 8 - 10 mm, 1 - 2 cm, 2 - 4 cm, or 4 - 10 cm. The width of the device (e.g., the diameter of the tube, or the diameter of the first and second members), may be from about 0.1 - 0.2 mm, 0.2 - 0.4 mm, 0.4 - 0.6 mm, 0.6 - 0.8 mm, 0.8 - 1.0 mm, 1 - 2 mm, 2 - 4 mm, 4-6 mm, 6 - 8 mm, 8 - 10 mm, 1-2 cm, 2 - 4 cm, or 4 - 10 cm. The device may be shaped and sized for injection (e.g., less than about 4 mm long and less than about 0.5 mm in diameter, e.g., to fit through at least one of a needle having a size from about 30 gauge to about 15 gauge or a cannula having a size from about 30 gauge to about 15 gauge, preferably to fit through a less than 22-gauge cannula). When such devices are prepared for implantation within the vitreous of the eye, the device may not exceed about 7 mm in any direction, so that the device can be inserted through a less than 7 mm incision. Thus, the devices may not exceed 7 mm in height or 3 mm in diameter.

The tube wall is preferably sufficiently thick to allow the tube to retain its shape in the absence of any other material. The thickness of the tube walls may range between about 0.01 mm and about 1.0 mm. The tube wall may be from about 0.01 - 0.02 mm, 0.02 - 0.04 mm, 0.04 - 0.06 mm, 0.06 - 0.08 mm, 0.08 - 1.0 mm, 1 - 2 mm, 2 - 4 mm, or 4 - 10 mm. Exemplary diameters for the tube of the device include 0.011" +/- 0.001" for the inner diameter and 0.0145" +/- 0.001" for the outer diameter. Exemplary diameters for the tube of the device also include 0.0061 +/-0.001" for the inner diameter and 0.0145" +/- 0.001" for the outer diameter. Exemplary diameters for the tube of the device also include 0.016" +/- 0.001" for the inner diameter and 0.018" +/- 0.001" for the outer diameter.

At least a portion of the shell is permeable to the beneficial substance. "Permeable" denotes that the shell allows an effective amount of the beneficial substance to exit the device. Certain parts of the shell may be impermeable to the beneficial substance. The term "impermeable", as used herein, means that the layer will not allow passage of the beneficial substance at a rate required to obtain the desired local or systemic physiological or pharmacological effect, during the period when the device delivers an effective amount of the beneficial substance to the patient. The impermeable region may have a permeability for the beneficial substance of less than 10%, 5%, 2%, 1%, 0.5%, 0.2%, 0.1%, 0.05%, 0.02%, or 0.01% of the permeability of the permeable region.

The tube may be permeable to the beneficial substance. The first member may be permeable to the beneficial substance. The second member may be permeable to the beneficial substance. The tube may be impermeable and one or both of the members are permeable. To promote greater release of the beneficial substance, the tube may be made of a permeable material. For instance, the tube, first member, and second member may all be permeable.

The permeability of a portion of the shell can be affected by its thickness. An impermeable member should be thick enough not to release a significant amount of beneficial substance, relative to a permeable region of the device. The thickness of an impermeable member can be, for example, between about 0.01 and about 2 mm, preferably between about 0.01 and about 0.5 mm, most preferably between about 0.01 and about 0.2 mm. A permeable member should be thick enough to contain the carrier particles in the tube, yet not so thick as to prevent release of an effective amount of the beneficial substance. The thickness of the permeable member can be, for example, between about 0.01 and about 2 mm, preferably between about 0.01 and about 0.5 mm, most preferably between about 0.01 and about 0.2 mm.

At least a portion of the shell may be porous and the beneficial substance can exit the shell through the pores. The pores should be small enough that the carrier particles do not pass through the pores in any substantial amount.

Preferably, the shell is essentially insoluble in body fluids which the material will come in contact.

The shell ma ybe substantially non-biodegradable. The shell may not substantially biodegrade in a biological environment prior to release of at least 90%, 95%, or 99% of the beneficial substance. The shell may substantially biodegrade in a biological environment after release of at least 90%, 95%, or 99% of the beneficial substance.

The shell may comprise a polymer. In particular, the tube, first member, and/or second member may be polymeric. Generally speaking, suitable biocompatible polymers for use in the subject devices include, but are not limited to, poly(vinyl acetate) (PVAC), poly(caprolactone) (PCL), ethylene vinyl acetate polymer (EVA), poly(ethylene glycol) (PEG), poly(vinyl alcohol) (PVA), poly(lactic acid) (PLA), poly(glycolic acid) (PGA), poly(lactic-co-glycolic acid) (PLGA), polyalkyl cyanoacrylate, polyurethane, nylons, or copolymers thereof. In polymers including lactic acid monomers, the lactic acid may be D-, L- (e.g., poly-L-lactic acid (PLLA)), or any mixture of D- and L- isomers. The polymer may be polyimide. The polymer may be PLGA that comprises lactic acid (L) and glycolic acid (G) monomers in a ratio of about 95% L and 5% G. The percentage of L may range between 80-97%. The percentage of G may range between 3-20%. The polymer may be heat curable, radiation curable, light (including ultraviolet) curable, evaporation curable, or curable by catalysis. The polymer may be silicone, such as a silicone rubber, polydimethylsiloxane, or silicone-carbonate copolymer.

Certain polymers, like PVA, can be made more or less permeable by altering the degree of polymer cross-linking. Some polymers may be permeable or impermeable depending on the relative characteristics of the polymer and the drug in the drug core. For instance, a given polymer may be permeable to a small molecule but impermeable to an antibody.

Exemplary polymers suitable for construction of permeable portions of the shell include PVA and PEG.

Exemplary polymers suitable for construction of impermeable portions of the shell include nylons, polyurethane, EVA, polyalkyl cyanoacrylate, poly(tetrafluoroethylene) (PTFE), polycarbonate (PC), poly(methyl methacrylate) (PMMA), high grades of ethylene vinyl acetate (EVA) (e.g., 9% vinyl, content), poly(lactic-co-glycolic acid) (PLGA), and polyvinyl alcohol (PVA), especially cross-linked PVA.

The shell may comprise metal, such as gold, platinum, and (surgical) stainless steel. For instance, the tube may be made of metal. The metal portion of the shell may be impermeable to the beneficial substance. The metal is preferably biocompatible. The metal may be biodegradable. The biocompatible and/or biodegradable metal alloy may comprise one or more of Fe (iron), Mg (magnesium), Mn (manganese), Pd (palladium), Co (cobalt), Al (aluminum), W (tungsten), B (boron), C (carbon), S (sulfur), Si (silicon), Li (lithium), Zr (zirconium), Ca (calcium), Y (yttrium), Zn (zinc). Exemplary biodegradable metals are described in H. Hermawan "Biodegradable Metals" SpringerBriefs in Materials 2012 p. 13-22 and Moravej and Martovani, "Biodegradable Metals for Cardiovascular Stent Application: Interests and New Opportunities" Int J Mol Sci. 2011; 12(7): 4250-4270.

The shell may comprise silicon (for instance, elemental silicon) or silica. A silicon or silica shell may be biodegradable.

The materials for the tube, first member, and second member can be chosen to achieve the desired rate of release for the beneficial substance. For instance, a "low-flow" example of the device is illustrated in cross-section in Figure 4. The device 400 comprises a tube 402 made of an impermeable material and a first member 404 made of an impermeable material. The second member 406 is made of a permeable material. The particles 408 are contained inside the device 400. The particles 408 initially release the beneficial substance 410 into the interior of the device. The beneficial substance 410 then diffuses through the permeable second member 406 to exit the device. No substantial amount of the beneficial substance exits the device through the tube 402 or first member 404. As an example, the tube may comprise impermeable poly(dl-lactide-co-glycolide) PLGA, the first member may be made of an impermeable substance such as silicone, and the second member may comprise permeable poly(vinyl alcohol) (PVA).

In contrast, Figure 5 illustrates a "high-flow" example of the device, in cross-section. The device 500 comprises a tube 502 made of an impermeable material. The first member 504 and second member 506 are both made of permeable material. The particles 508 are contained inside the device 500. The particles 508 initially release the beneficial substance 510 into the interior of the device. The beneficial substance 510 then diffuses through the permeable first and second members 504 and 506 to exit the device. No substantial amount of the beneficial substance exits the device through the tube 502. As an example, the tube may comprise impermeable poly(dl-lactide-co-glycolide) PLGA, and the first and second members may comprise permeable poly(vinyl alcohol) (PVA).

The beneficial substance diffuses in the direction of lower chemical potential, i.e., toward the exterior surface of the device. Release of the beneficial substance from the device is controlled by several factors. It is influenced by the beneficial substance's dissolution rate, rate of release from the pores of the carrier material, and passage through the shell. The device shape, size, and materials can be chosen to achieve a desired release rate of the beneficial substance. Thus, the release rate may be determined primarily by dissolution of the beneficial substance. Also, the release rate may be determined primarily by release of the beneficial substance from pores of the carrier material. Further, the release rate may be determined primarily by the permeability of the shell. The release rate may be significantly affected by any two or all three of these steps.

The rate of diffusion of the beneficial substance through the device's shell may be determined, for instance, via diffusion cell studies carried out under sink conditions. In diffusion cell studies carried out under sink conditions, the concentration of drug in the receptor compartment is essentially zero when compared to the high concentration in the donor compartment.

It will be appreciated that a material may be permeable to a drug and also substantially control the rate at which the drug diffuses or otherwise passes through the material. Consequently, a permeable portion of the shell may also be release-rate-limiting or release-rate-controlling, and the permeability of such a membrane may be one of the most significant factors controlling the release rate for a device.

The device may release the beneficial substance at a rate that is essentially constant over time (i.e., zero-order kinetics). Zero-order release is desirable when the goal is to maintain a substantially constant amount of the beneficial substance to the patient over a sustained period.

### B. Characteristics of the carrier material

The carrier material holds and gradually releases a beneficial substance, such as a drug. The beneficial substance resides in small pores in the carrier material. The carrier material is a plurality of particles enclosed by the device's shell. Each of the carrier particles releases beneficial substance into the interior of the device, and from there the beneficial substance then exits the shell.

The particles of the device, measured at the largest diameter, may have a d10 in the range 1-5 microns (meaning 10% of the particles in the device have a diameter below a number in the range of 1-5 microns), a d50 in the range 5-10 microns (meaning 50% of the particles in the device have a diameter below a number in the range of 5-10 microns), and a d90 in the range of 10-20 microns (meaning 90% of the particle in the device have a diameter below a number in the range of 10-20 microns). The d10 may be 1-5 microns, 1-3 microns, or 3-5 microns. The d50 may be 5-7 microns, 6-8 microns, 7-9 microns, or 8-10 microns. The d90 may be 10-15 microns or 15-20 microns. The particles of the device, measured at the largest diameter, may have an average size of about 1 to about 500 microns, such as about 5 to 500 microns, about 5 to about 100 microns, or about 5 to about 20 microns. For instance, greater than 60%, greater than 70%, greater than 80% or greater than 90% of the particles have a particle size of from 1-20 microns, preferably 5-15 microns, measured at the largest dimension. The particles may have an average particle size between 1 and 20 microns such as between 5-15 microns or about 15 microns, about 16 microns, about 17 microns, about 18 microns, about 19 microns.

The carrier may comprise a semiconductor material such as semiconductor silicon. Examples of additional materials that may be used as porous carrier materials are germanium, ceramics, metal oxides, bone phosphate, phosphates of calcium (e.g., hydroxyapatite), other inorganic phosphates, carbon black, carbonates, sulfates, aluminates, borates, aluminosilicates, magnesium oxide, calcium oxide, iron oxides, zirconium oxides, titanium oxides, and other comparable materials.

The device may comprise a silicon-based carrier material such as elemental silicon, silicon dioxide (silica), silicon monoxide, silicates (compounds containing a silicon-bearing anion, e.g., SiF₆²⁻, Si₂O₇⁶⁻, or SiO₄⁴⁻), or any combination of such materials. The carrier material may be, for example, semiconducting silicon, elemental silicon, polycrystalline silicon, or amorphous silicon. The silicon may be undoped, or may be doped (for example with phosphorus). The carrier material may be silicon carbide or silicon nitride. Preferably, the carrier material comprises a complete or partial framework of elemental silicon and that framework is substantially or fully covered by a silicon dioxide surface layer. II is also preferred that the carrier material is entirely or substantially entirely silica. The silicon-based carrier material may be synthetic amorphous silica. The silicon-based carrier material may be fumed silica.

The carrier material may comprise silica, such as greater than about 50% silica, greater than about 60 wt% silica, greater than about 70 wt% silica, greater than about 80 wt% silica, greater than about 90 wt% silica, greater than about 95 wt% silica, greater than 99 wt% silica, or even greater than 99.9 wt% silica. Porous silica may be purchased from suppliers such as Grace Davison (and sold under the trademark Davisil), Silicycle, and Macherey-Nagel.

The carrier material may comprise elemental silicon, greater than 60 wt% silicon, greater than 70 wt% silicon, greater than 80 wt% silicon, greater than 90 wt% silicon, or even greater than 95 wt% silicon. Silicon may be purchased from suppliers such as Vesta Ceramics.

Purity of the silicon-based material can be quantitatively assessed using techniques such as Energy Dispersive X-ray Analysis, X-ray fluorescence, Inductively Coupled Optical Emission Spectroscopy or Glow Discharge Mass Spectroscopy.

The based carrier material may a porous, amorphous solid or a porous, crystalline solid. For example, silicon-based carrier material may comprise elemental silicon or compounds thereof, e.g., silicon dioxide or silicates, in an amorphous form. The elemental silicon or compounds thereof may be present in a crystalline form. The carrier material may comprise amorphous silica and/or amorphous silicon. The silicon-based material may be greater than about 60 wt% amorphous, greater than about 70 wt% amorphous, greater than about 80 wt% amorphous, greater than about 90 wt% amorphous, greater than about 92 wt% amorphous, greater than about 95 wt% amorphous, greater than about 99 wt% amorphous, or even greater than 99.9 wt% amorphous. The amorphous silica may be fumed silica. The amorphous silica may be synthetic amorphous silica.

X-ray diffraction analysis can be used to identify crystalline phases of silicon-based material. Powder diffraction can be taken, for example, on a Scintag PAD-X diffractometer, e.g., equipped with a liquid nitrogen cooled germanium solid state detector using Cu K-alpha radiation. The carrier material may have a porosity of about 30% to about 95%, about 30% to about 90%, or about 60% to about 80%. Porosity, as used herein, is a measure of the void spaces in a material, and is a fraction of the volume of voids over the total volume of the material. The carrier material may have a porosity of at least about 10%, at least about 20%, at least about 30%, at least about 40%, at least about 50%, at least about 60%, at least about 70%, at least about 80%, or even at least about 90%. The porosity may be greater than about 40%, such as greater than about 50%, greater than about 60%, or even greater than about 70%.

The carrier material of the devices may have a surface area to weight ratio selected from about 20 m²/g to about 2000 m²/g, such as from about 20 m²/g to about 1000 m²/g, or even from about 100 m²/g to about 300 m²/g. The surface area may be greater than about 200 m²/g, greater than about 250 m²/g or greater than about 300 m²/g. The surface area may be about 200 m²/g.

The beneficial substance may be distributed to a pore depth from the surface of the material of at least about 10 microns, at least about 20 microns, at least about 30 microns, at least about 40 microns, at least about 50 microns, at least about 60 microns, at least about 70 microns, at least about 80 microns, at least about 90 microns, at least about 100 microns, at least about 110 microns, at least about 120 microns, at least about 130 micron, at least about 140 microns or at least about 150 microns. The beneficial substance may be distributed in the pores of the carrier material substantially uniformly.

The beneficial substance may be loaded into the carrier material to a depth which is measured as a ratio of the depth to which the beneficial substance penetrates the carrier material to the total width of the carrier material. The beneficial substance may be distributed to a depth of at least about 10% into the carrier material, to at least about 20% into the carrier material, at least about 30% into the carrier material, at least about 40% into the carrier material, at least about 50% into the carrier material, or at least about 60% into the carrier material.

Quantification of gross loading may be achieved by a number of analytic methods, for example, gravimetric, EDX (energy-dispersive analysis by x-rays), Fourier transform infra-red (FTIR) or Raman spectroscopy of the pharmaceutical composition or by UV spectrophotometry, titrimetric analysis, HPLC or mass spectroscopy of the eluted therapeutic agent in solution. Quantification of the uniformity of loading may be obtained by compositional techniques that are capable of spatial resolution such as cross-sectional EDX, Auger depth profiling, micro-Raman and micro-FTIR.

The carrier material preferably comprises pores that can receive a beneficial substance. Microporous carrier (pore size less than 2 nm), mesoporous carrier (pore size 2-50 nm) and macroporous carrier (pore size >50 nm) are all suitable carrier materials. The average pore size of the carrier material may be selected from 2-50 nm, such as from about 5 to about 40 nm, from about 15 to about 40 nm, such as about 20 to about 30 nm. The average pore size may be selected from about 2 to about 15 nm, such as about 5 to about 10 nm. The average pore size may be about 30 nm. For instance, greater than 50% of the pores of the carrier material have a pore size from 2-50 nm, greater than 60% of the pores of the carrier material have a pore size from 2-50 nm, greater than 70% of the pores of the carrier material have a pore size from 2-50 nm, greater than 80% of the pores of the carrier material have a pore size from 2-50 nm, or even greater than 90% of the pores of the carrier material have a pore size from 2-50 nm.

The carrier material may comprise porous silicon dioxide, such as mesoporous silicon dioxide or amorphous silica, such as fumed silica.

The carrier material may have a population of pores with a well-defined pore size, i.e., the distribution of pore sizes for the carrier material falls within a defined range. A well-defined population of pores may have about 50% to about 99% of the pore sizes within about 1 nm to 15 nm of the average pore size for that population, preferably within about 10 nm, about 5 nm, or even within 3 nm or 2 nm of the average pore size for that population. In certain such aspects, greater than about 50%, greater than about 60%, greater than about 70%, greater than about 80%, greater than about 90%, or even greater than about 95% of the pores of the carrier material have pore sizes within the specified range. Similarly, a population of pores with a well-defined pore size can be a population in which greater than about 50%, greater than about 60%, greater than about 70%, greater than about 80%, greater than about 90%, or even greater than about 95% of the pores have pore sizes within 20%, preferably within 15%, 10%, or even 5% of the average pore size for that population.

Pore (e.g., mesopore) size distribution can be quantified using established analytical methods such as gas adsorption, high resolution scanning electron microscopy, nuclear magnetic resonance cryoporosimetry and differential scanning calorimetry.

A population of pores with a well-defined pore size can be a population for which the standard deviation of the pore sizes is less than 20%, preferably less than 15%, less than 10%, or even less than 5% of the average pore size for that population.

The pore size may be preselected to the dimensional characteristics of the beneficial substance to control the release rate of the beneficial substance in a biological system. Typically, pore sizes that are too small preclude loading of the beneficial substance, while oversized pores do not interact with the beneficial substance sufficiently strongly to exert the desired control over the rate of release. For example, the average pore diameter for a carrier material may be selected from larger pores, e.g., 15 nm to 40 nm, for high molecular weight molecules, e.g., 200,000-500,000 amu, and smaller pores, e.g., 2 nm to 10 nm, for molecules of a lower molecular weight, e.g., 10,000-50,0000 amu. For instance, average pore sizes of about 6 nm in diameter may be suitable for molecules of molecular weight around 14,000 to 15,000 amu, such as about 14,700 amu. Average pore sizes of about 10 nm in diameter may be selected for molecules of molecular weight around 45,000 to 50,000 amu, such as about 48,000 amu. Average pore sizes of about 25-30 nm in diameter may be selected for molecules of molecular weight around 150,000 amu.

The pore size may be preselected to be adapted to the molecular radius of the beneficial substance to control the release rate of the beneficial substance in a biological system. For instance, average pore sizes of about 25 nm to about 40 nm in diameter may be suitable for molecules with a largest molecular radius from about 6 nm to about 8 nm. Molecular radii may be calculated by any suitable method such as by using the physical dimensions of the molecule based on the X-ray crystallography data or using the hydrodynamic radius which represents the solution state size of the molecule. As the solution state calculation is dependant upon the nature of the solution in which the calculation is made, it may be preferable for some measurements to use the physical dimensions of the molecule based on X-ray crystallography data. As used herein, the largest molecular radius reflects half of the largest dimension of the therapeutic agent.

The average pore diameter may be selected to limit the aggregation of molecules, e.g., proteins, within a pore. It would be advantageous to prevent biomolecules, such as proteins, from aggregating in a device as this is believed to impede the controlled release of molecules into a biological system. Therefore, a pore that, due to the relationship between its size and the size of a biomolecule, allows, for example, only one biomolecule to enter the pore at any one time will be preferable to a pore that allows multiple biomolecules to enter the pore together and aggregate within the pore. Multiple biomolecules (e.g., proteins) may be loaded into a pore, but due to the depth of the pore, the proteins distributed throughout this depth of the pore will aggregate to a lesser extent. For instance, a pore may have a diameter slightly larger than the diameter of a protein inside the pore. In this case, the pore's narrow diameter can constrain the arrangement of proteins, decreasing aggregation.

The carrier material may comprise two or more different materials with different properties (e.g., pore sizes, particle diameters, or surface characteristics), each preselected to be adapted to a different beneficial substance. For example, two different carrier materials may be admixed, one with a first population of pores whose pore size is adapted to a first beneficial substance, the other with a second population of pores whose pore size is adapted to a second beneficial substance. The device may comprise a first population of carrier particles having a first population of pores whose pore size is adapted to a first beneficial substance, and a second population of carrier particles having a second population of pores whose pore size is adapted to a second beneficial substance. Also, a particle may comprise a single material that has two or more well-defined populations of pores, e.g., wherein the carrier material is made by a molecular templating technique, wherein the characteristics of the pores are preselected for two or more beneficial substances, e.g., two beneficial substances with different molecular radii. Thus, the carrier material may deliver two or more beneficial substances in the controlled manner described herein. Here, the loading of the beneficial substances is preferably ordered from largest to smallest agent, so that the largest agent selectively adsorbs into the largest pores (i.e., it does not fit into the smaller pores), so that the larger pores do not adsorb smaller agents.

In case the carrier material has two or more distinct well-defined populations of pores (e.g., the distinct pore populations are substantially non-overlapping), the differences between the properties of the different populations of pores are preferably selected to limit the adsorption of each different beneficial substance to a certain population of pores. The average pore size of the two or more distinct well-defined pore populations may be selected to limit the adsorption of the larger beneficial substance into smaller pores. The average pore size differential may be defined as the difference between the average pore sizes for the different populations of pores in the carrier material. For example, an average pore size differential of at least 10 nm could indicate that the carrier material may comprise at least two populations of pores whose average pore sizes differ ("average pore size differential") by at least 10 nm., e.g., the composition may comprise two pore populations having average pore sizes of 10 nm and 20 nm, three populations of pores with average pore sizes of 10 nm, 20 nm, and 30 nm, or four populations of pores with average pore sizes of 10 nm, 20 nm, 30 nm, and 40 nm. The average pore size differential is preferably at least about 5 nm, at least about 10 nm, at least 15 nm, at least about 20 nm, or at least about 30 nm. The two or more well-defined pore populations may have distinct average pore sizes, such that the average pore sizes of any two populations differ by at least 20%, preferably at least 30%, 40%, or even 50% of the smaller average pore size.

The walls of the carrier material that separate the pores may have an average width of less than 5 nm, such as about 4.8 nm, about 4.6 nm, about 4.4 nm, about 4.2 nm, about 4.0 nm, about 3.8 nm, about 3.6 nm, about 3.4 nm, about 3.2 nm, about 3.0 nm, about 2.8 nm, or even about 2.6 nm. The walls of the carrier material that separate the pores may have an average width of less than about 3 nm, such as about 2.8 nm, about 2.6 nm, about 2.4 nm, about 2.2 nm, about 2.0 nm, about 1.8 nm, about 1.6 nm, about 1.4 nm, about 1.2 nm, about 1.0 nm, or even about 0.8 nm.

Dimensionality and morphology of the device can be measured, for example, by Transmission Electron Microscopy (TEM) using a 2000 JEOL electron microscope operating, for example, at 200 keV. Samples for TEM can be prepared by dispensing a large number of porous carrier materials onto a holey carbon film on a metal grid, via a dilute slurry.

The pores of the carrier material may define space having a volume of about 0.1 mL/g to about 5 mL/g of the carrier material. The pore volume may be about 0.2 mL/g to about 3 mL/g, such as about 0.4 mL/g to about 2.5 mL/g, such as about 1.0 mL/g to about 2.5 mL/g.

The load level of the carrier material may be up to 70%, such as up to 40% by weight based on the combined weight of the carrier material and the beneficial substance. The load level is calculated by dividing the weight of the loaded beneficial substance by the combined weight of the loaded therapeutic agent and carrier material and multiplying by 100. The load level of the carrier material may be greater than 10%, such as greater than 15%, greater than 20%, greater than 25%, greater than 30%, greater than 35%, greater than 40%, greater than 45% or greater than 50%. The load level of the carrier material may less than 5%. The load level may be between about 5% and about 10%. The load level of the carrier material may be between about 10% and about 20%, between about 20% and about 30%, between about 30% and about 40%, between about 40% and about 50%, or between about 50% and about 60% by weight.

The load volume of the devices described herein may be evaluated in terms of the volume of the pores in the porous material being occupied by the beneficial substance. The percentage of the maximum loading capacity that is occupied by the beneficial substance (that is, the percentage of the total volume of the pores in the porous carrier material that is occupied by the beneficial substance) for carrier materials may be from about 30% to about 100%, such as from about 50% to about 90%. For any given carrier material, this value may be determined by dividing the volume of the beneficial substance taken up during loading by the void volume of the carrier material prior to loading and multiplying by one hundred.

The carrier particles, measured at the largest diameter, may have an average size of about 1 to about 500 microns, such as about 5 to about 100 microns. At least 80%, 90%, 99%, or even 100% of the particles in the device, measured at the largest diameter, may be about 1 to about 500 microns, such as about 5 to about 500 microns, or about 2 to about 100 microns.

In order to increase the rate of loading of the beneficial substance into the particles, it may be advantageous to use relatively small particles. As smaller particles have pores with less depth for the beneficial substance to penetrate, the amount of time needed to load the particles may be reduced. This may be particularly advantageous when the pore diameters are similar in dimensions to the molecular diameters or size of the therapeutic agents. Smaller particles may be from 1-20 microns, such as about 10-20 microns, e.g., about 15-20 microns, measured at the largest dimension.

In some aspects, greater than 60%, greater than 70%, greater than 80% or greater than 90% of the particles have a particle size of from 1-20 microns, preferably 5-15 microns, measured at the largest dimension. The particles may have an average particle size between 1 and 20 microns such as between 5-15 microns or about 15 microns, about 16 microns, about 17 microns, about 18 microns, about 19 microns.

Particle size distribution, including the mean particle diameter can be measured, for example, using a Malvern Particle Size Analyzer, Model Mastersizer, from Malvern Instruments, UK. A helium-neon gas laser beam may be projected through an optical cell containing a suspension of the carrier material. Light rays striking the carrier material are scattered through angles which are inversely proportional to the particle size. The photodetector array measures the light intensity at several predetermined angles and electrical signals proportional to the measured light flux values are then processed by a microcomputer system against a scatter pattern predicted from the refractive indices of the sample carrier material and aqueous dispersant.

Methods of preparing suitable carrier materials, including those described above, may be found in International Application WO 2012/061377.

The device may also comprise one or more pharmaceutically acceptable excipients. The excipient may be a filler, binder, diluent, buffering agent, moistening agent, preservative, stabilizer, flavoring agent, dye, coloring agent, disintegrating agent, or surfactant. A buffering agent may be used to tailor the drug release rate by creating a micro-environment pH in the device. The pH can affect the dissolution rate of the beneficial substance or the permeability of the shell for the beneficial substance, thereby affecting the overall release rate. A surfactant may be used to adjust the charge, lipophilicity or hydrophilicity of the carrier, such as to enhance wettability of poorly soluble or hydrophobic compositions. Some examples of materials which can serve as pharmaceutically acceptable excipients include: (1) sugars, such as lactose, glucose and sucrose; (2) starches, such as corn starch and potato starch; (3) cellulose, and its derivatives, such as sodium carboxymethyl cellulose, ethyl cellulose and cellulose acetate; (4) powdered tragacanth; (5) malt; (6) gelatin; (7) talc; (8) hydrophobic materials such as cocoa butter, suppository waxes, and the like; (9) oils, such as peanut oil, cottonseed oil, safflower oil, sesame oil, olive oil, corn oil and soybean oil; (10) glycols, such as propylene glycol; (11) polyols, such as glycerin, sorbitol, mannitol and polyethylene glycol; (12) esters, such as ethyl oleate and ethyl laurate; (13) agar; (14) buffering agents, such as magnesium hydroxide and aluminum hydroxide; (15) alginic acid; and (16) other non-toxic compatible substances employed in pharmaceutical formulations. The excipient may be disposed within pores of the carrier material. Also, the excipient may be outside the particles and inside the device's shell. For instance, the particles may be suspended in solution and/or form a slurry, and the excipient may be in the solution.

### C. Beneficial substances deliverable by the device

The device can store and deliver a therapeutically effective amount of a beneficial substance. Preferably, the beneficial substance is a therapeutic. As used herein, the term "therapeutic" encompasses the active molecule as well as salts of the active molecule. The therapeutic may be, for example, a drug or a prodrug.

The beneficial substance may be selected from any agent useful in the treatment or prevention of diseases. The beneficial substance may be selected from small molecule therapeutic agents, i.e., compounds with molecular weights less than 1000 amu. The beneficial substance may be selected from large molecules with molecular weight equal to or greater than 1000 amu. The beneficial substance of the invention may be a biomolecule. Biomolecules, as used herein, refer to any molecule that is produced by a living organism, including large polymeric molecules such as proteins, polysaccharides, and nucleic acids as well as small molecules such as primary metabolites, secondary metabolites, and natural products or synthetic variations thereof. In particular, proteins such as antibodies, ligands, and enzymes may be used as beneficial substances in the devices described herein. In particular, the biomolecules for use in the device may have molecular weights ranging from about 10,000 amu to about 500,000 amu.

The beneficial substance may be a protein such as an antibody. The antibody may be a monoclonal antibody. The antibody may be, for instance, an antigen-binding portion of a full-length antibody, e.g., a Fab fragment or a single chain variable fragment. Particular therapeutic antibodies that may be delivered by the device include ranibizumab and bevacizumab.

Polynucleotides that may be administered using the devices herein include DNA, RNA, and analogs of DNA and RNA. For example, the polynucleotides may include 2'O-Me nucleotides or dideoxynucleotides. The polynucleotides may encode proteins for gene therapy, or may be designed to reduce expression of a target gene via an antisense pathway.

The beneficial substance may have a molecular weight between 10,000 and 50,000 amu, between 50,000 and 100,000 amu or between 100,000 and 150,000 amu. The beneficial substance may be a protein with a molecular weight between 5,000 amu and 200,000 amu, such as about 10,000 to about 150,000 amu.

The size of a beneficial substance may alternatively be characterized by the molecular radius, which may be determined, for example, through X-ray crystallographic analysis or by hydrodynamic radius. The beneficial substance may be a protein, e.g., with a molecular radius selected from 0.5 nm to 20 nm such as about 0.5 nm to 10 nm, even from about 1 to 8 nm.

The pore size of the carrier material can be selected based at least in part on the size of the beneficial substance. A beneficial substance with molecular radius from 1 to 2.5 nm may be advantageously used with a carrier material with a minimum pore radius of from 4.5 to 5.8 nm. A beneficial substance with a molecular radius of 7 nm may be advantageously used with a carrier material with a minimum pore radius of from 11 to 13 nm, such as about 12 nm. Additional discussion of selecting an appropriate size of pore for a given beneficial substance may be found, for example, in International Application WO 2012/061377.

The carrier particles may be loaded with a liquid drug. Advantageously, carrier particles loaded with a liquid drug can be easier to handle than the liquid drug itself. Thus, the devices herein can ease the manufacturing process for controlled release devices that deliver liquid drugs. The liquid drug may comprise a carboxylic acid moiety. The liquid beneficial substance in the device may be prostaglandin or a prostaglandin analog. For instance, the liquid drug may be the free acid form of latanoprost. As another example, the liquid drug may be a free acid form of travoprost. The liquid drug may be prostacyclin or a prostacyclin analog such as treprostinil, iloprost, or beraprost. The liquid may comprise a fat-soluble vitamin such as Vitamin E.

The beneficial substance may be prone to decomposition and/or inactivation, and the carrier material may reduce decomposition/inactivation of the beneficial substance. The beneficial substance can be inactivated, for example, by degradation or unfolding/denaturation. For instance, the beneficial substance might be prone to inactivation during the process of curing the first or second member (e.g., with heat or ultraviolet light) and the carrier material reduces this inactivation. For instance, the carrier material may block the agent that inactivates the beneficial substance (e.g., by absorbing the wavelength of light used for curing). As another example, the carrier may stabilize a beneficial substance against the effects of the agent that inactivates the beneficial substance, e.g., by stabilizing the beneficial substance in an active conformation and restricting protein unfolding or degradation. The beneficial substance may experience at least twice, five times, ten times, 20 times, 50, times or 100 times as much inactivation (e.g., degradation or unfolding) without the carrier material as the beneficial substance within the carrier material under the same conditions during production of the device.

The beneficial substance within the carrier material may have a half-life at room temperature that is at least twice, five times, ten times, 20 times, 50, times or 100 times the half-life of the beneficial substance without the carrier material under the same conditions. The beneficial substance within the carrier material may have a shelf-life at room temperature that is at least twice, five times, ten times, 20 times, 50, times or 100 times the shelf life of the beneficial substance without the carrier material under the same conditions. The beneficial substance within the carrier material may be stable at 25 °C for at least 15 days, 1 month, 2 months, 3 months, 6 months, at least 1 year, at least 1.5 years, at least 2 years, at least 2.5 years, at least 3 years or at least 4 years. Stability may be assessed, for example, by high performance size exclusion chromatography (HPSEC) or by comparing the biological activity of the stored biomolecule-loaded devices against a sample of freshly prepared biomolecule-loaded devices or against the activity of the devices as measured prior to storage. Preferably, at the end of the storage period, the activity of the stored devices is at least 75%, at least 80%, at least 85%, at least 90%, at least 95%, at least 98%, at least 99%, at least 99.5%, at least 99.8%, or even at least 99.9% of the activity of the corresponding freshly prepared devices. Accordingly, this disclosure contemplates methods of treatment wherein biomolecule-loaded devices are stored at 25 °C for at least 6 months, at least 1 year, at least 1.5 years, at least 2 years, at least 2.5 years, at least 3 years or at least 4 years prior to administering the devices to a patient. The degradation-sensitive beneficial substance may be a biomolecule, such as a protein, including an antibody.

The device may comprises two or more beneficial substances. For instance, the device may comprise two populations of particles, each population loaded with one beneficial substance. Alternatively, a single particle may comprise two or more beneficial substances. In some such cases, the single particle has a population of larger pores and a population of smaller pores, and each population of pores contains one of the beneficial substances. Carriers with two populations of pores are described above in Section B.

Many different beneficial substances may be incorporated into the devices described above (e.g., such as devices comprising a shell and porous silicon-based carrier particles). For example, suitable drugs include steroids, alpha receptor agonists, beta receptor antagonists, carbonic anhydrase inhibitors, adrenergic agents, physiologically active peptides and/or proteins, antineoplastic agents, antibiotics, analgesics, anti-inflammatory agents, muscle relaxants, antiepileptics, anti-ulcerative agents, anti-allergic agents, cardiotonics, anti-arrhythmic agents, vasodilators, antihypertensive agents, anti-diabetic agents, anti-hyperlipidemics, anticoagulants, hemolytic agents, antituberculous agents, hormones, narcotic antagonists, osteoclastic suppressants, osteogenic promoters, angiogenesis suppressors, antibacterials, non-steroidal anti-inflammatory drugs (NSAIDs), glucocorticoids or other anti-inflammatory corticosteroids, alkaloid analgesics, such as opioid analgesics, antivirals, such as nucleoside antivirals or a non-nucleoside antivirals, anti-benign prostatic hypertrophy (BPH) agents, anti-fungal compounds, antiproliferative compounds, anti-glaucoma compounds, immunomodulatory compounds, cell transport/mobility impeding agents, cytokines, pegylated agents, alpha-blockers, anti-androgens, anti-cholinergic agents, purinergic agents, dopaminergic agents, local anesthetics, vanilloids, nitrous oxide inhibitors, anti-apoptotic agents, macrophage activation inhibitors, antimetabolites, neuroprotectants, calcium channel blockers, gamma-aminobutyric acid (GABA) antagonists, alpha agonists, anti-psychotic agents, tyrosine kinase inhibitors, nucleoside compounds, and nucleotide compounds, and analogs, derivatives, pharmaceutically acceptable salts, esters, prodrugs, and protected forms thereof.

Suitable NSAIDs for use in the devices described herein (e.g., devices comprising a shell and porous silicon-based carrier particles) include diclofenac, etoldolac, fenoprofen, floctafenine, flurbiprofen, ibuprofen, indoprofen, ketoprofen, ketorolac, lomoxicam, morazone, naproxen, perisoxal, pirprofen, pranoprofen, suprofen, suxibuzone, tropesin, ximoprofen, zaltoprofen, zileuton, and zomepirac, and analogs, derivatives, pharmaceutically acceptable salts, esters, prodrugs, and protected forms thereof.

Suitable carbonic anhydrase inhibitors for use in the devices described herein (e.g., devices comprising a shell and porous silicon-based carrier particles) include brinzolamide, acetazolamide, methazolamide, dichlorphenamide, ethoxzolamide, and dorzolamide, and analogs, derivatives, pharmaceutically acceptable salts, esters, prodrugs, and protected forms thereof.

Suitable adrenergic agents for use in the devices described herein (e.g., devices comprising a shell and porous silicon-based carrier particles) include brimonidine, apraclonidine, bunazosin, levobetaxolol, levobunalol, carteolol, isoprenaline, fenoterol, metipranolol, and clenbuterol, and analogs, derivatives, pharmaceutically acceptable salts, esters, prodrugs, and protected forms thereof.

Suitable alpha receptor agonists for use in the devices described herein (e.g., devices comprising a shell and porous silicon-based carrier particles) include brimonidine and analogs, derivatives, pharmaceutically acceptable salts, esters, prodrugs, and protected forms thereof.

Suitable beta receptor antagonists for use in the devices described herein (e.g., devices comprising a shell and porous silicon-based carrier particles) include atenolol, betaxolol, and timolol, and analogs, derivatives, pharmaceutically acceptable salts, esters, prodrugs, and protected forms thereof.

Suitable antiviral agents for use in the devices described herein (e.g., devices comprising a shell and porous silicon-based carrier particles) include nevirapine and analogs, derivatives, pharmaceutically acceptable salts, esters, prodrugs, and protected forms thereof.

Suitable alkaloid analgesics for use in the devices described herein (e.g., devices comprising a shell and porous silicon-based carrier particles) include desmorphine, dezocine, dihydromorphine, eptazocine, ethylmorphine, glafenine, hydromorphone, isoladol, ketobenidone, p-lactophetide, levorphanol, moptazinol, metazocin, metopon, morphine, nalbuphine, nalmefene, nalorphine, naloxone, norlevorphanol, normorphine, oxmorphone, pentazocine, phenperidine, phenylramidol, tramadol, and viminol, and analogs, derivatives, pharmaceutically acceptable salts, esters, prodrugs, and protected forms thereof.

Suitable glucocorticoids for use in the devices described herein (e.g., devices comprising a shell and porous silicon-based carrier particles) include 21-acetoxypregnenolone, alclometasone, algestone, anacortave acetate, amcinonide, beclomethasone, betamethasone, budesonide, chloroprednisone, clobetasol, clobetasone, clocortolone, cloprednol, corticosterone, cortisone, cortivazol, deflazacort, desonide, desoximetasone, diflorasone, diflucortolone, difuprednate, enoxolone, fluazacort, flucloronide, flumethasone, flunisolide, fluocinolone acetonide, fluocinonide, flucloronide, flumethasone, flunisolide, fluocortin butyl, fluocortolone, fluorometholone, fluperolone acetate, fluprednisolone, flurandrenolide, fluticasone propionate, hydrocortamate, hydrocortisone, meprednisone, methylprednisolone, paramethasone, prednisolone, prednisolone 21-diethylaminoacetate, fluprednidene acetate, formocortal, loteprednol etabonate, medrysone, mometasone furoate, prednicarbate, prednisolone, prednisolone 25-diethylaminoacetate, prednisolone sodium phosphate, prednisone, prednival, prednylidene, triamcinolone, triamcinolone acetonide, triamcinolone benetonide, and triamcinolone hexacetonide, and analogs, derivatives, pharmaceutically acceptable salts, esters, prodrugs, and protected forms thereof.

Other suitable steroids for use in the devices described herein (e.g., devices comprising a shell and porous silicon-based carrier particles) include halcinonide, halbetasol propionate, halometasone, halopredone acetate, isoflupredone, loteprednol etabonate, mazipredone, rimexolone, and tixocortol, and analogs, derivatives, pharmaceutically acceptable salts, esters, prodrugs, and protected forms thereof.

Suitable BPH drugs for use in the devices described herein (e.g., devices comprising a shell and porous silicon-based carrier particles) include finasteride and osaterone, and analogs, derivatives, pharmaceutically acceptable salts, esters, prodrugs, and protected forms thereof.

Suitable antineoplastic compounds for use in the devices described herein (e.g., devices comprising a shell and porous silicon-based carrier particles) include adriamycin, alitretinoin (9-cis-retinoic acid); bleomycins, including bleomycin A; capecitabine (5'-deoxy-5-fluoro-cytidine); carubicin; chlorozotocin, chromomycins, including chromomycin A3, cladribine; colchicine, cytarabine; daunorubicin; demecolcine, denopterin, docetaxel, doxyifluridine, doxorubicin; dromostanolone, edatrexate, enocitabine, epirubicin, epitiostanol, estramustine; etoposide; floxuridine, fludarabine, 5-fluorouracil, formestane, gemcitabine; irinotecan; lentinan, lonidamine, melengestrol, melphalan; menogaril, methotrexate; mitolactol; nogalamycin; nordihydroguaiaretic acid, olivomycins such as olivomycin A, paclitaxel; pentostatin; pirarubicin, plicamycin, porfiromycin, prednimustine, puromycin; ranimustine, ristocetins such as ristocetin A; temozolamide; teniposide; tomudex; topotecan; tubercidin, ubenimax, valrubicin (N-trifluoroacetyladriamycin-14-valerate), vinorelbine, vinblastine, vindesine, vinorelbine, and zorubicin and analogs, derivatives, pharmaceutically acceptable salts, esters, prodrugs, and protected forms thereof.

Suitable antibacterial compounds for use in the devices described herein (e.g., devices comprising a shell and porous silicon-based carrier particles) include capreomycins, including capreomycin IA, capreomycin IB, capreomycin IIA and capreomycin IIB; carbomycins, including carbomycin A; carumonam; cefaclor, cefadroxil, cefamandole, cefatrizine, cefazedone, cefazolin, cefbuperazone, cefcapene pivoxil, cefclidin, cefdinir, cefditoren, cefime, ceftamet, cefmenoxime, cefmetzole, cefminox, cefodizime, cefonicid, cefoperazone, ceforanide, cefotaxime, cefotetan, cefotiam, cefoxitin, cefpimizole, cefpiramide, cefpirome, cefprozil, cefroxadine, cefsulodin, ceftazidime, cefteram, ceftezole, ceftibuten, ceftiofur, ceftizoxime, ceftriaxone, cefuroxime, cefuzonam, cephalexin, cephalogycin, cephaloridine, cephalosporin C, cephalothin, cephapirin, cephamycins, such as cephamycin C, cephradine, chlortetracycline; chlarithromycin, clindamycin, clometocillin, clomocycline, cloxacillin, cyclacillin, danofloxacin, demeclocyclin, destomycin A, dicloxacillin, dirithromycin, doxycyclin, epicillin, erythromycin A, ethanbutol, fenbenicillin, flomoxef, florfenicol, floxacillin, flumequine, fortimicin A, fortimicin B, forfomycin, foraltadone, fusidic acid, gentamycin, glyconiazide, guamecycline, hetacillin, idarubicin, imipenem, isepamicin, josamycin, kanamycin, leumycins such as leumycin A1, lincomycin, lomefloxacin, loracarbef, lymecycline, meropenam, metampicillin, methacycline, methicillin, mezlocillin, micronomicin, midecamycins such as midecamycin A1, mikamycin, minocycline, mitomycins such as mitomycin C, moxalactam, mupirocin, nafcillin, netilicin, norcardians such as norcardian A, oleandomycin, oxytetracycline, panipenam, pazufloxacin, penamecillin, penicillins such as penicillin G, penicillin N and penicillin O, penillic acid, pentylpenicillin, peplomycin, phenethicillin, pipacyclin, piperacilin, pirlimycin, pivampicillin, pivcefalexin, porfiromycin, propiallin, quinacillin, ribostamycin, rifabutin, rifamide, rifampin, rifamycin SV, rifapentine, rifaximin, ritipenem, rekitamycin, rolitetracycline, rosaramicin, roxithromycin, sancycline, sisomicin, sparfloxacin, spectinomycin, streptozocin, sulbenicillin, sultamicillin, talampicillin, teicoplanin, temocillin, tetracyclin, thostrepton, tiamulin, ticarcillin, tigemonam, tilmicosin, tobramycin, tropospectromycin, trovafloxacin, tylosin, and vancomycin, and analogs, derivatives, pharmaceutically acceptable salts, esters, prodrugs, and protected forms thereof.

Suitable anti-fungal compounds for use in the devices described herein (e.g., devices comprising a shell and porous silicon-based carrier particles) include fluconazole and analogs, derivatives, pharmaceutically acceptable salts, esters, prodrugs, and protected forms thereof.

Suitable immunological response modifiers for use in the devices described herein (e.g., devices comprising a shell and porous silicon-based carrier particles) include muramyl dipeptide and analogs, derivatives, pharmaceutically acceptable salts, esters, prodrugs, and protected forms thereof.

Suitable peptides and proteins for use in the devices described herein (e.g., devices comprising a shell and porous silicon-based carrier particles) include insulin, growth hormones, insulin related growth factor, heat shock proteins, and analogs, derivatives, pharmaceutically acceptable salts, esters, prodrugs, and protected forms thereof.

Suitable anesthetics and pain killing agents for use in the devices described herein (e.g., devices comprising a shell and porous silicon-based carrier particles) include lidocaine, benzodiazepam, and analogs, derivatives, pharmaceutically acceptable salts, esters, prodrugs, and protected forms thereof.

Suitable cell transport/mobility impending agents for use in the devices described herein (e.g., devices comprising a shell and porous silicon-based carrier particles) include cytochalasin B and analogs, derivatives, pharmaceutically acceptable salts, esters, prodrugs, and protected forms thereof.

Antiproliferative/antimitotic drugs and prodrugs suitable for use in the devices described herein (e.g., devices comprising a shell and porous silicon-based carrier particles) include natural products such as vinca alkaloids (e.g., vinblastine, vincristine, and vinorelbine), paclitaxel, epidipodophyllotoxins (e.g., etoposide, teniposide), antibiotics (e.g., actinomycins, daunorubicin, doxorubicin and idarubicin), anthracyclines, mitoxantrone, bleomycins, plicamycin (mithramycin) and mitomycin, enzymes (e.g., L-asparaginase); antiplatelet prodrugs; antiproliferative/antimitotic alkylating prodrugs such as nitrogen mustards (mechlorethamine, cyclophosphamide and analogs, melphalan, chlorambucil), ethylenimines and methylmelamines (hexamethylmelamine and thiotepa), alkyl sulfonates-busulfan, nitrosoureas (carmustine (BCNU) and analogs, streptozocin), triazenes, dacarbazine (DTIC); antiproliferative/antimitotic antimetabolites such as folic acid analogs (methotrexate), pyrimidine analogs (fluorouracil, floxuridine, and cytarabine), purine analogs and related inhibitors (mercaptopurine, thioguanine, pentostatin and 2-chlorodeoxyadenosine (cladribine); platinum coordination complexes (cisplatin, carboplatin), procarbazine, hydroxyurea, mitotane, aminoglutethimide; hormones (e.g., estrogen, progestin); anticoagulants (e.g., heparin, synthetic heparin salts and other inhibitors of thrombin); fibrinolytic prodrugs such as tissue plasminogen activator, streptokinase and urokinase, aspirin, dipyridamole, ticlopidine, clopidogrel, abciximab; antimigratories; antisecretories (breveldin); anti-inflammatory agents such as corticosteroids (cortisol, cortisone, fludrocortisone, flucinolone, prednisone, prednisolone, methylprednisolone, triamcinolone, betamethasone, and dexamethasone), NSAIDS (salicylic acid and derivatives, aspirin, acetaminophen, indole and indene acetic acids (indomethacin, sulindac and etodalac), heteroaryl acetic acids (tolmetin, diclofenac, and ketorolac), arylpropionic acids (e.g., ibuprofen and derivatives), anthranilic acids (mefenamic acid, and meclofenamic acid), enolic acids (piroxicam, tenoxicam, phenylbutazone, and oxyphenthatrazone), nabumetone, gold compounds (auranofin, aurothioglucose, gold sodium thiomalate), and 6-mannose phosphate; immunosuppressives (e.g., cyclosporine, tacrolimus (FK-506), sirolimus (rapamycin), azathioprine, and mycophenolate mofetil); angiogenic agents such as vascular endothelial growth factor (VEGF), fibroblast growth factor (FGF); angiotensin receptor blocker; nitric oxide donors; anti-sense oligonucleotides and combinations thereof; cell cycle inhibitors, mTOR inhibitors, growth factor signal transduction kinase inhibitors, neovascularization inhibitors, angiogenesis inhibitors, and apoptosis inhibitors, and analogs, derivatives, pharmaceutically acceptable salts, esters, prodrugs, and protected forms thereof.

Suitable antiviral agents for use in the devices described herein (e.g., devices comprising a shell and porous silicon-based carrier particles) include acyclovir, azidouridine, anismoycin, amantadine, bromovinyldeoxusidine, chlorovinyldeoxusidine, cytarabine, delavirdine, didanosine, deoxynojirimycin, dideoxycytidine, dideoxyinosine, dideoxynucleoside, desciclovir, deoxyacyclovir, efavirenz, enviroxime, fiacitabine, foscamet, fialuridine, fluorothymidine, floxuridine, ganciclovir, hypericin, idoxuridine, interferon, interleukin, isethionate, nevirapine, pentamidine, ribavirin, rimantadine, stavudine, sargramostin, suramin, trichosanthin, tribromothymidine, trichlorothymidine, trifluorothymidine, trisodium phosphomonoformate, vidarabine, zidoviridine, zalcitabine and 3-azido-3-deoxythymidine and analogs, derivatives, pharmaceutically acceptable salts, esters, prodrugs, and protected forms thereof.

Other suitable antiviral agents for use in the devices described herein (e.g., devices comprising a shell and porous silicon-based carrier particles) include 2',3'-dideoxyadenosine (ddA), 2',3'-dideoxyguanosine (ddG), 2',3'-dideoxycytidine (ddC), 2',3'-dideoxythymidine (ddT), 2'3'-dideoxy-dideoxythymidine (d4T), 2'-deoxy-3'-thia-cytosine (3TC or lamivudime), 2',3'-dideoxy-2'-fluoroadenosine, 2',3'-dideoxy-2'-fluoroinosine, 2',3'-dideoxy-2'-fluorothymidine, 2',3'-dideoxy-2'-fluorocytosine, 2'3'-dideoxy-2',3'-didehydro-2'-fluorothymidine (Fd4T), 2'3'-dideoxy-2'-beta-fluoroadenosine (F-ddA), 2'3'-dideoxy-2'-beta-fluoro-inosine (F-ddI), and 2',3'-dideoxy-2'-beta-flurocytosine (F-ddC). The antiviral agent may be selected from trisodium phosphomonoformate, ganciclovir, trifluorothymidine, acyclovir, 3'-azido-3'-thymidine (AZT), dideoxyinosine (ddI), and idoxuridine and analogs, derivatives, pharmaceutically acceptable salts, esters, prodrugs, and protected forms thereof.

Beneficial substances suitable for administration to the eye and its surrounding tissues, using the devices described herein (e.g., devices comprising a shell and porous silicon-based carrier particles), to produce a local or a systemic physiologic or pharmacologic beneficial effect include neuroprotectants such as nimodipine and related compounds; antibiotics such as tetracycline, chlortetracycline, bacitracin, neomycin, polymyxin, gramicidin, oxytetracycline, chloramphenicol, gentamycin, and erythromycin; antibacterials such as sulfonamides, sulfacetamide, sulfamethizole, and sulfisoxazole; antivirals, including idoxuridine; and other antibacterial agents such as nitrofurazone and sodium propionate; antiallergenics such as antazoline, methapyriline, chlorpheniramine, pyrilamine, and prophenpyridamine; antiinflammatories such as hydrocortisone, hydrocortisone acetate, dexamethasone 21-phosphate, fluocinolone, medrysone, methylprednisolone, prednisolone 21-phosphate, prednisolone acetate, fluoromethalone, betamethasone, and triamcinolone; decongestants such as phenylephrine, naphazoline, and tetrahydrazoline; miotics and anti-cholinesterase such as pilocarpine, eserine salicylate, carbachol, di-isopropyl fluorophosphate, phospholine iodine, and demecarium bromide; mydriatics such as atropine sulfate, cyclopentolate, homatropine, scopolamine, tropicamide, eucatropine, and hydroxyamphetamine; sympathomimetics such as epinephrine; and analogs, derivatives, pharmaceutically acceptable salts, esters, prodrugs, and protected forms thereof; and prodrugs such as those described in Design of Prodrugs, edited by Hans Bundgaard, Elsevier Scientific Publishing Co., Amsterdam, 1985. Reference may be made to any standard pharmaceutical textbook such as Remington's Pharmaceutical Sciences for the identification of other agents.

Prodrugs are generally compounds that, under physiological conditions, are converted into therapeutically active agents in a patient's body. A common method for making a prodrug is to include selected moieties, such as esters, that are hydrolyzed under physiological conditions to convert the prodrug to an active biological moiety. The prodrug may also be converted by an enzymatic activity of the host animal. Prodrugs are typically formed by chemical modification of a biologically active moiety. Conventional procedures for the selection and preparation of suitable prodrug derivatives are described, for example, in Design of Prodrugs, ed. H. Bundgaard, Elsevier, 1985.

Any pharmaceutically acceptable form of such a compound may be employed as a beneficial substance, i.e., the free base or a pharmaceutically acceptable salt or ester thereof. Pharmaceutically acceptable salts, for instance, include sulfate, lactate, acetate, stearate, hydrochloride, tartrate, maleate, and the like.

### 2. Methods of preparation

The devices described herein may be made in a wide variety of ways, portions of which are described above in Section 1. In preferred aspects, the device is prepared by filling a tube with carrier particles and enclosing the ends of the tube.

Carrier particles are prepared or provided. The carrier particles may be made porous by introducing pores into non-porous material. This may be done, for instance, by anodizing a solid such as silicon to produce parallel pores. Anodization may be performed together with etching, e.g., wet etching or dry etching. Thus, the pores may be formed by electrochemical etching.

The carrier particles may be porous when they are first formed. For example, in sol-gel synthesis, a solution is induced to form a porous network or gel of polymers. Sol-gel synthesis can be used, for example, to make porous silica. Porous silica can be chemically reduced to silicon by treatment with, for instance, magnesium vapor. The magnesium vapor method and related methods for reducing silica are described in International Application WO/2012/114126.

Part or all of a large body of carrier material may be made porous, and the porous region may then be milled into small particles. The particles may be formed of a non-porous material, and then pores may be introduced into the particles.

In forming carrier particles, a mesh may be used to select particles of the desired size.

The porous silicon-based carrier material may be prepared by flame hydrolysis of silicon tetrachloride in an oxy-hydrogen flame.

Carrier particles may be loaded with a beneficial substance before or after the particles are assembled into the device.

A tube may be produced or provided. The tube may be extruded, e.g., from a polymeric mass. Commercially available extruders include the Randcastle model RCP-0250 Microtruder (Randcastle Extrusion Systems, Cedar Grove, N.J.), and its associated heaters, controllers, and the like. Exemplary extruders are also disclosed, for example, in U.S. Pat. Nos. 5,569,429, 5,518,672, and 5,486,328. An extruder can include an exit port that establishes a cross-sectional shape of the extruded matter. The tube may be cured after extrusion. The tube may be coextruded with the carrier particle composition inside the tube. The tube may be formed in the absence of carrier particles, and carrier particles are inserted into the formed tube. The tube may be segmented into a plurality of tubular segments. Segmentation may employ shears, slicing blades, or any other technique.

The tube is filled with carrier particles. The tube may be several multiples of the length of the finished device, and the tube is cut to the proper length before or after being filled with carrier particles. The particles may be dry when they are placed in the tube, and may have the consistency of a powder or granulate. The particles may be in solution when they are placed in the tube, and may have the consistency of a slurry. The slurry may be drawn into the tube with capillary action or pushed in using a syringe, for example. Preferably , the carrier particles are loaded with the beneficial substance when they are placed in the tube. However, the particles may also be loaded with drug after this step, e.g., when they are in the tube but before members are added to close the tube, or even after one or both members are added.

The first member and second member are added to the first and second ends of the tube to enclose the particles. One or both of the members may be formed *in situ* on the end or ends of the tube. An amount of polymer solution may be applied to the end of the tube. For instance, one end of the tube can be dipped in a polymer solution one or more times, so that a film of the polymer will form on the end of the tube. Alternatively, the polymer solution may be applied to the end of the tube by dropping, spraying, brushing or other means. Once the polymer is in contact with the tube, the polymer can be cured (hardened by cross-linking) on the end of the tube. Polymers can be cured, e.g., using heat, radiation, light (including ultraviolet light or visible light such as blue light), evaporation, and catalyzation. Curing with light in the visible or near-visible ranges (e.g., of ultraviolet or blue wavelengths) sometimes avoids inactivation of the beneficial substance that can result from harsher curing techniques. Curing may be performed using an intense light source, such as a tuned laser or the like. Each polymer can be cured using one or more suitable curing techniques, and numerous examples are known in the art. For instance, PVA may be cured with (for instance) ultraviolet light, infrared light, and/or by heating it in an oven. The desired thickness of the first or second member may be obtained by applying more than one coat of a polymer. Each coat may be dried and/or cured prior to applying the next coat.

The device shell may comprise heat-cured PVA. In particular, a heat-cured first member and/or second member may be formed by applying a PVA solution to the first end of the tube and then heating the PVA. The PVA may be heated, e.g., at a temperature in the range of 60-120°C, e.g., 80°C, for at least 2 hours, preferably at least 4 hours, e.g., 5 hours. Heating may be performed, for example, in an oven or other heating element.

The device and/or the components of the device may be sterilized. The heat-resistant portions of the device may be heat-sterilized, e.g., by autoclaving. Other methods of sterilizing the device or its components include sterilization with radiation, ultraviolet light, immersion in alcohol, or filtration. A heat-sensitive component (such as a beneficial substance, including a biomolecule) may be filter-sterilized. All heat sterilization of the device or its components may take place prior to addition of the heat-sensitive component to the device. For example, the particles and/or the shell may be heat-sterilized before the beneficial substance is added to the device.

The device may be packaged, such as by preloading a needle of appropriate gauge with the device and enclosing the assembly in a suitable package for shipment to an end user.

### 3. Methods of use

The present disclosure also provides methods for treating a patient to obtain a desired local or systemic physiological or pharmacological effect. These methods include administering the disclosed devices to the patient and allowing the beneficial substance to pass through the device to come in direct contact with the patient. The device can be administered for a sufficient period of time and under conditions to allow treatment of the disease state of concern. The patient may be a mammalian organism, and preferably the patient is a human.

The device may be inserted into a desired location in the patient's body. For instance, the device may be injected or surgically implanted into the patient's body. When the beneficial substance acts on the eye, the device can gradually release the beneficial substance to the eye, avoiding painful repeated administrations of a different formulation of the beneficial substance. Accordingly, the device can be surgically implanted into the eye of the patient, for example the vitreous of the eye, under the retina, and onto the sclera. The device can also be inserted into numerous other locations in the body, including administration that is subcutaneous, intramuscular, intraperitoneal, intranasal, dermal, into the brain, including intracranial and intradural, into the joints, including ankles, knees, hips, shoulders, elbows, wrists, directly into tumors, and the like. The device can also be administered orally.

The device may be administered to a patient by injection. Injection may use, for example, a standard gauge hypodermic needle, such as about a 30 gauge to about a 12 gauge needle, or a needle ranging in inside diameter from about 0.0055 inches to about 0.0850 inches. An injectable device can also be administered through, for example, an arthroscope, catheter, or other medical device.

For localized drug delivery, the devices may be surgically implanted at or near the site of action. This is the case for devices as described herein, used in treating ocular conditions, primary tumors, rheumatic and arthritic conditions, and chronic pain, for instance.

For systemic relief, the devices may be implanted subcutaneously, intramuscularly, intraarterially, intrathecally, or intraperitoneally, for example. This is the case when devices are to give sustained systemic levels and avoid premature metabolism. In addition, the devices may be administered orally.

The devices described herein may be particularly suitable for treating ocular conditions such as glaucoma, proliferative vitreoretinopathy, macular edema, including diabetic macular edema, age-related macular degeneration, diabetic retinopathy, uveitis, ocular neovascularization, and ocular infection. The devices may also be particularly suitable for use as an ocular device in treating patients, both human and for veterinarian use, suffering from ocular histoplasmosis, wherein the device may be surgically implanted within the vitreous of the eye.

The device may contain one or more drugs that reduce the risk of mother to child transmission of viral infections. Examples of viral infections include HIV, Bowenoid Papulosis, Chickenpox, Childhood HIV Disease, Human Cowpox, Hepatitis C, Dengue, Enteroviral, Epidermodysplasia Verruciformis, Erythema Infectiosum (Fifth Disease), Giant Condylomata Acuminata of Buschke and Lowenstein, Hand-Foot-and-Mouth Disease, Herpes Simplex, Herpes Virus 6, Herpes Zoster, Kaposi Varicelliform Eruption, Rubeola Measles, Milker's Nodules, Molluscum Contagiosum, Monkeypox, Orf, Roseola Infantum, Rubella, Smallpox, Viral Hemorrhagic Fevers, Genital Warts, and Nongenital Warts.

The device may contain an antiviral agent that inhibits or reduces HIV infection or susceptibility to HIV infection. The device may be used to treat mammalian organisms infected with HIV and AIDS-related opportunistic infections such as cytomegalovirus infections, toxoplasmosis, pneumocystis carinii, and mycobacterium avium intercellular.

The device may contain one or more drugs that treat pulmonary arterial hypertension.

The device may be used to provide a controlled and sustained release of agents effective in obtaining a desired local or systemic physiological or pharmacological effect relating at least to the following areas: treatment of cancerous primary tumors, (e.g., glioblastoma); inhibition of neovascularization, including ocular neovascularization; edema, including ocular edema; inflammation, including ocular inflammation; chronic pain; arthritis; rheumatic conditions; hormonal deficiencies such as diabetes and dwarfism; and modification of the immune response such as in the prevention of transplant rejection and in cancer therapy. A wide variety of other disease states may also be prevented or treated using the delivery devices herein. Such disease states are known by those of ordinary skill in the art. For those not skilled in the art, reference may be made to Goodman and Gilman, The Pharmacological Basis of Therapeutics, 8th Ed., Pergamon Press, NY, 1990; and Remington's Pharmaceutical Sciences, 18th Ed., Mack Publishing Co., Easton, Pa., 1990.

The device (e.g., a device comprising a shell enclosing porous silicon-based carrier particles) may deliver ganciclovir to the eye for the treatment of cytomegalovirus (CMV) retinitis. The device (e.g., a device comprising a shell enclosing porous silicon-based carrier particles) may deliver fluocinolone acetonide to the eye for the treatment of ocular neovascularization, macular edema, wet or dry age-related macular degeneration, retinal vein occlusion, or posterior uveitis. The device (e.g., a device comprising a shell enclosing porous silicon-based carrier particles) may deliver bevacizumab or ranibizumab for the treatment of neovascularization, including ocular neovascularization, for example when caused by cancer, macular degeneration (especially wet age-related macular degeneration), diabetic retinopathy, neovascular glaucoma, macular edema, or retinopathy. The device (e.g., a device comprising a shell enclosing porous silicon-based carrier particles) may deliver latanoprost to the eye for treatment of ocular hypertension and/or glaucoma.

The devices herein may release the beneficial substance over a sustained time period when immersed in simulated body fluid or when administered to a patient. For instance, the device may release an effective amount of the beneficial substance for between 1 week - 1 year, 2 weeks - 1 year, 1 month - 1 year, 2 months - 1 year, 3 months - 1 year, or 6 months - 1 year. The device may release the effective amount of the beneficial substance for 1 month - 2 yeas, 2 months - 2 years, 3 months - 2 years, or 6 months - 2 years.

The invention now being generally described, it will be more readily understood by reference to the following examples, which are included merely for purposes of illustration of certain aspects and embodiments of the present invention, and are not intended to limit the invention.

### EXAMPLES

### Example 1: Method of making silicon particles

Mesoporous silicon flakes of approximately 5 mm² were pooled from membranes fabricated by anodization of 5-20 mohm cm resistivity silicon wafers. The electrolyte composition was a fixed blend (1:1 by volume) of 40% hydrofluoric acid and methanol. Flakes were extensively washed in an ethanol-water mixture, dried, and then subjected to a sequence of rotor milling and jet milling to produce a powder of defined size distribution, prior to thermal oxidation in air at 800 °C for 3 hours. The final target size distribution (d10 in the range of 1-5 microns, d50 in the range of 5-10 microns, and d90 in the range of 10-20 micron) was then achieved by sieving at small batch size or a sedimentation technique at larger batch size.

## Claims

1. A device for delivering a beneficial substance to an eye of a patient, comprising a shell and a plurality of particles disposed within the shell, the particles comprising a porous silicon-based carrier material and a beneficial substance disposed within pores of the carrier material, and the shell comprising at least one portion that is permeable to the beneficial substance, wherein the beneficial substance is selected from the group consisting of neuroprotectants, antibacterials, antiallergenics, antiinflammatories, decongestants, miotics, mydriatics, and sympathomimetics.

2. The device of claim 1, wherein the shell comprises a polymer.

3. The device of claim 1 or 2, wherein the shell comprises:
(a) a tube having first and second ends, optionally wherein the tube has a substantially cylindrical shape;
(b) a first member positioned at the first end; and
(c) a second member positioned at the second end;
wherein the particles are inside the tube and the first and second members retain the particles in the tube.

4. The device of claim 3, wherein the tube is impermeable to the beneficial substance, optionally wherein the tube comprises poly(lactic-co-glycolic acid) (PLGA).

5. The device of any of claim 3 or 4, wherein the first member and second member are permeable to the beneficial substance, optionally wherein the first member and second member optionally comprise poly(vinyl alcohol) (PVA).

6. The device of any of claims 3-5, wherein the first member is permeable to the beneficial substance, optionally wherein the first member optionally comprises poly(vinyl alcohol) (PVA); and the second member is impermeable to the beneficial substance, optionally wherein the second member comprises silicone.

7. The device of any of claims 3-6, wherein the tube has a length of between 1 and 4 mm, and/or optionally the tube has a diameter of between 0.2 and 0.5 mm.

8. The device of any of claims 1-7, wherein the carrier material is mesoporous, the carrier material has a porosity in the range of about 50% to about 80%; the pores of the carrier material are substantially parallel; or the particles of the carrier material have a d50 in the range of 5-10 microns.

9. The device of any of claims 1-8, wherein the carrier material comprises elemental silicon, further optionally wherein the carrier material comprises silica, optionally wherein the carrier material comprises anodized silicon.

10. The device of any of claims 1-9, wherein the carrier material is resorbable or bio-erodible.

11. A method of making a device, comprising:
(a) providing a tube having first and second ends;
(b) inserting a plurality of particles into the tube, wherein the particles comprise a porous silicon-based carrier material;
(c) adding a first member to the first end of the tube before or after inserting the plurality of particles into the tube; and
(d) adding a second member to the second end to the tube before or after inserting the plurality of particles into the tube,
wherein at least one of the tube, the first member, and the second member is permeable to the beneficial substance.

12. The method of claim 11, wherein the particles comprise a beneficial substance disposed within pores of the carrier material; or the method further comprises contacting the device to a beneficial substance and allowing the beneficial substance to enter pores of the carrier material.

13. The method of claim 11 or 12, wherein adding the first member comprises contacting the first end of the tube to a polymer solution and curing the polymer at the first end, thereby forming a first member on the first end of the tube; or adding the second member comprises contacting the second end of the tube to a polymer solution and curing the polymer at the second end, thereby forming a second member on the second end of the tube.

## Patentansprüche

1. Vorrichtung zur Abgabe einer heilsamen Substanz in ein Auge eines Patienten, umfassend eine Schale und eine Vielzahl von Partikeln, die in der Schale disponiert sind, wobei die Partikel ein poröses siliziumbasiertes Trägermaterial und eine heilsame Substanz, disponiert in den Poren des Trägermaterials umfassen, und wobei die Schale mindestens einen Teil umfasst, der für die heilsame Substanz permeabel ist, wobei die heilsame Substanz aus der Gruppe bestehend aus Neuroprotektoren, antibakteriellen Mitteln, Antiallergika, Entzündungshemmern, Abschwellern, Miotika, Mydriatika und Sympathomimetika ausgewählt ist.

2. Vorrichtung nach Anspruch 1, wobei die Schale ein Polymer umfasst.

3. Vorrichtung nach Anspruch 1 oder 2, wobei die Schale umfasst:
(a) ein Röhrchen mit einem ersten und zweiten Ende, wobei das Röhrchen gegebenenfalls eine im Wesentlichen zylindrische Form hat;
(b) ein erstes Element, das am ersten Ende positioniert ist; und
(c) ein zweites Element, das am zweiten Ende positioniert ist,
wobei die Partikel im Inneren des Röhrchens sind und das erste und das zweite Element die Partikel im Röhrchen halten.

4. Vorrichtung nach Anspruch 3, wobei das Röhrchen für die heilsame Substanz impermeabel ist, wobei das Röhrchen gegebenenfalls Poly(lactid-co-Glykolsäure) (PLGA) umfasst.

5. Vorrichtung nach Anspruch 3 oder 4, wobei das erste Element und das zweite Element für die heilsame Substanz permeabel sind, wobei das erste Element und das zweite Element gegebenenfalls Poly(vinylalkohol) (PVA) umfassen.

6. Vorrichtung nach einem der Ansprüche 3 bis 5, wobei das erste Element für die heilsame Substanz permeabel ist, wobei das erste Element gegebenenfalls Poly(vinylalkohol) (PVA) umfasst; und das zweite Element für die heilsame Substanz impermeabel ist, wobei das zweite Element gegebenenfalls Silikon umfasst.

7. Vorrichtung nach einem der Ansprüche 3 bis 6, wobei das Röhrchen eine Länge zwischen 1 mm und 4 mm hat, und/oder das Röhrchen gegebenenfalls einen Durchmesser zwischen 0,2 und 0,5 mm hat.

8. Vorrichtung nach einem der Ansprüche 1 bis 7, wobei das Trägermaterial mesoporös ist, das Trägermaterial eine Porosität im Bereich von etwa 50% bis etwa 80% hat; die Poren des Trägermaterial im Wesentlichen parallel angeordnet sind; oder die Partikel des Trägermaterials einen d50 im Bereich 5-10 Mikrometer haben.

9. Vorrichtung nach einem der Ansprüche 1 bis 8, wobei das Trägermaterial elementares Silizium umfasst, wobei weiterhin das Trägermaterial gegebenenfalls Silica umfasst, wobei das Trägermaterial gegebenenfalls eloxiertes Silizium umfasst.

10. Vorrichtung nach einem der Ansprüche 1 bis 9, wobei das Trägermaterial resorbierbar oder biologisch abbaubar ist.

11. Verfahren zur Herstellung einer Vorrichtung umfassend:
(a) Bereitstellen eines Röhrchens mit einem ersten und einem zweiten Ende,
(b) Einführen einer Vielzahl von Partikel in das Röhrchen, wobei die Partikel ein poröses siliziumbasiertes Trägermaterial umfassen;
(c) Zufügen eines ersten Elements an das erste Ende des Röhrchens vor oder nach dem Einführen der Vielzahl von Partikeln in das Röhrchen; und
(d) Zufügen eines zweiten Elements an das zweite Ende des Röhrchens vor
oder nach dem Einführen der Vielzahl von Partikeln in das Röhrchen, wobei mindestens eines, ausgewählt aus Röhrchen, erstem Element und zweitem Element für die heilsame Substanz permeabel ist.

12. Verfahren nach Anspruch 11, wobei die Partikel eine heilsame Substanz disponiert in den Poren des Trägermaterials umfassen; oder wobei das Verfahren weiterhin das Inkontaktbringen der Vorrichtung mit einer heilsamen Substanz und das Zulassen des Eintritts der heilsamen Substanz in die Poren des Trägermaterials umfasst.

13. Verfahren nach Anspruch 11 oder 12, wobei das Zufügen des ersten Elements das Inkontaktbringen des ersten Endes des Röhrchens mit einer Polymerlösung und das Aushärten des Polymers am ersten Ende umfasst, wodurch ein erstes Element am ersten Ende des Röhrchens gebildet wird; oder das Zufügen des zweiten Elements das Inkontaktbringen des zweiten Endes des Röhrchens mit einer Polymerlösung und das Aushärten des Polymers am zweiten Ende umfasst wodurch ein zweites Element am zweiten Ende des Röhrchens gebildet wird.

## Revendications

1. Dispositif pour délivrer une substance bénéfique à un œil d'un patient, comprenant une enveloppe et une pluralité de particules disposées à l'intérieur de l'enveloppe, les particules comprenant un matériau de support poreux à base de silicium et une substance bénéfique disposée à l'intérieur de pores du matériau de support, et l'enveloppe comprenant au moins une partie qui est perméable à la substance bénéfique, dans lequel la substance bénéfique est choisie dans le groupe constitué par les neuroprotecteurs, les antibactériens, les antiallergiques, les anti-inflammatoires, les décongestionnants, les miotiques, les mydriatiques, et les sympathomimétiques.

2. Dispositif selon la revendication 1, dans lequel l'enveloppe comprend un polymère.

3. Dispositif selon la revendication 1 ou 2, dans lequel l'enveloppe comprend :
(a) un tube ayant des première et deuxième extrémités, optionnellement dans lequel le tube a une forme pratiquement cylindrique ;
(b) un premier élément positionné à la première extrémité ; et
(c) un deuxième élément positionné à la deuxième extrémité ;
dans lequel les particules sont à l'intérieur du tube et les premier et deuxième éléments retiennent les particules dans le tube.

4. Dispositif selon la revendication 3, dans lequel le tube est imperméable à la substance bénéfique, optionnellement dans lequel le tube comprend du poly(acide lactique-co-glycolique) (PLGA).

5. Dispositif selon la revendication 3 ou 4, dans lequel le premier élément et le deuxième élément sont perméables à la substance bénéfique, optionnellement dans lequel le premier élément et le deuxième élément comprennent optionnellement du poly(alcool vinylique) (PVA).

6. Dispositif selon l'une quelconque des revendications 3 à 5, dans lequel le premier élément est perméable à la substance bénéfique, optionnellement dans lequel le premier élément comprend optionnellement du poly(alcool vinylique) (PVA) ; et le deuxième élément est imperméable à la substance bénéfique, optionnellement dans lequel le deuxième élément comprend de la silicone.

7. Dispositif selon l'une quelconque des revendications 3 à 6, dans lequel le tube a une longueur comprise entre 1 et 4 mm, et/ou optionnellement le tube a un diamètre compris entre 0,2 et 0,5 mm.

8. Dispositif selon l'une quelconque des revendications 1 à 7, dans lequel le matériau de support est mésoporeux, le matériau de support a une porosité située dans la plage allant d'environ 50 % à environ 80 % ; les pores du matériau de support sont pratiquement parallèles ; ou les particules du matériau de support ont une d50 située dans la plage allant de 5 à 10 micromètres.

9. Dispositif selon l'une quelconque des revendications 1 à 8, dans lequel le matériau de support comprend du silicium élémentaire, en outre optionnellement dans lequel le matériau de support comprend de la silice, optionnellement dans lequel le matériau de support comprend du silicium anodisé.

10. Dispositif selon l'une quelconque des revendications 1 à 9, dans lequel le matériau de support est résorbable ou bio-érodable.

11. Méthode de fabrication d'un dispositif, comprenant:
(a) l'obtention d'un tube ayant des première et deuxième extrémités;
(b) l'insertion d'une pluralité de particules dans le tube, lesquelles particules comprennent un matériau de support poreux à base de silicium;
(c) l'addition d'un premier élément à la première extrémité du tube avant ou après l'insertion de la pluralité de particules dans le tube; et
(d) l'addition d'un deuxième élément à la deuxième extrémité du tube avant ou après l'insertion de la pluralité de particules dans le tube,
dans laquelle au moins l'un parmi le tube, le premier élément, et le deuxième élément, est perméable à la substance bénéfique.

12. Méthode selon la revendication 11, dans laquelle les particules comprennent une substance bénéfique disposée à l'intérieur de pores du matériau de support ; ou laquelle méthode comprend en outre la mise en contact du dispositif avec une substance bénéfique et le fait de laisser la substance bénéfique entrer dans des pores du matériau de support.

13. Méthode selon la revendication 11 ou 12, dans laquelle l'addition du premier élément comprend la mise en contact de la première extrémité du tube avec une solution de polymère et le durcissement du polymère à la première extrémité, ce qui forme ainsi un premier élément sur la première extrémité du tube ; ou l'addition du deuxième élément comprend la mise en contact de la deuxième extrémité du tube avec une solution de polymère et le durcissement du polymère à la deuxième extrémité, ce qui forme ainsi un deuxième élément sur la deuxième extrémité du tube.
